# EUROPEAN PATENT APPLICATION

(11) **EP 1 156 512 A1**
(43) Date of publication of application: **21.11.2001**
(21) Application number: 01111859.3
(22) Date of filing: 16.05.2001
(51) Int. Cl.: H01J 61/22, H01J 61/16

(54) **Discharge lamp for photodynamic therapy and photodynamic diagnosis**

(30) Priority: 19.05.2000 JP 2000148097
(71) Applicant: USHIO DENKI KABUSHIKI KAISYA, Tokyo-to, 100-0004 (JP)
(72) Inventor: Hiramoto, Tatsumi, Tokyo-to 153-0051 (JP); Higashi, Tadatoshi, Himeji-shi, Hyogo-ken 670-0940 (JP); Kimura, Makoto, Yokohama-shi, Kanagawa-ken 232-0071 (JP)
(74) Representative: Ebner von Eschenbach, Jennifer

(57) **Abstract**

The present invention provides a discharge lamp capable of effectively radiating a light suitable for a photosensitizer used in PDT and PDD, and relates to a discharge lamp which radiates a light suitable for the wavelength region of absorption of a photosensitizer having a relatively large absorption coefficient in the range of wavelength of 600n-800nm, and more specifically, the discharge lamp is filled with 0.1 µ mol/cm³ or more of any of the elements selected from the group of lithium (Li), sodium (Na), rubidium (Rb) and potassium (K) as an emitting element, and in addition, at least one or more rare gases selected from the group of neon (Ne), argon (Ar), krypton (Kr) and xenon (Xe) is also filled, and by filling such an emitting element, a light having the wavelength region of the main absorption within the range of the wavelength of 600nm-800nm can be radiated.

## Description

### BACKGROUND OF THE INVENTION

### 1. Field of the Invention

The present invention relates to a light source and a light source apparatus, which are utilized in photodynamic therapy and photodynamic diagnosis of a cancer, tumor or the like.

### 2. Description of the Related Art

As methods of diagnosis and therapy of a cancer, tumor or the like, photodynamic therapy (hereinafter referred to as PDT) and photodynamic diagnosis (hereinafter referred to as PDD) are used.

In the case of PDT, by either administering a certain kind of medicine to a living body, which functions as a photosensitizer, or applying a photosensitizer topically to an affected part of a cancer, tumor or the like, either the medicine itself, or a substance generated by internal metabolisms in the body is selectively accumulated in the affected part.

When a light of a specific wavelength is irradiated onto the accumulated part thereof, a photochemical reaction is occurred, and thereby either an active oxygen is generated, or a special radical is generated in the irradiated part thereof. The active oxygen thereof causes necrosis of the cells of the tissue in the body by oxidation thereof and the like, and as a result, a cancer, tumor or the like is remedied.

Further, as an active type, which is generated as a result of absorption of light by a photosensitizer, and particularly, a singlet oxygen to be generated is preferable, and therefore, the light energy to be irradiated must correspond to the wavelength of 800nm or less.

Further, as the light to be irradiated for generating the active oxygen and so forth, since a light of a wavelength region capable of permeating deeply inside the tissue of the body is required, the wavelength of approximately 600nm or more is considered to be necessary.

Such a method of treatment can also be applied to a viral verruca, an infectious water verruca or the like, in addition to a cancer or tumor.

Further, in the case of PDD, a medicine used as a photosensitizer, is one that absorbs mainly a light in the vicinity of 400nm, and thereby emits a red fluorescence. And then, by observing the fluorescence thereof, the affected part of a cancer or tumor in which the photosensitizer has selectively accumulated is diagnosed.

For example, Protoporphyrin IX, which is one of photosensitizes, reacts to a light in the region of the wavelength of 405nm, and thereby radiates the light of 635nm as fluorescence.

As a conventional PDT, PDD apparatus, for example, there is the one disclosed in USP4,556,057 (Japanese Patent Laid-open No. S59-40830). In the technology disclosed therein, a hematoporphyrin derivative is used as the photosensitizer, and a dye laser is used as a light source.

Furthermore, as a light source in the conventional PDT, a semiconductor laser, xenon lamp or the like is used.

Further, as a light source in the conventional PDD, a mercury lamp, xenon lamp or the like is mainly used.

However, because the diameter of the beam of laser light of the high-output laser, the dye laser or the like, which is mainly used as the light source for therapy (for PDT), is extremely small, there arises a problem that a long time irradiation is required when an irradiation to a large area is performed, which causes a great burden to a patient.

Further, there is also a problem that the apparatus is extremely large and lacks portability, which requires the patient to move to the place where the apparatus is installed.

Furthermore, there are also various other problems that the cost of the apparatus is high, that handling thereof is difficult, and that a specially-trained operator is needed to operate the apparatus.

Meanwhile, semiconductor lasers, light-emitting diodes or the like which are also being used as light sources, do not produce sufficient output. Thus, there arises a problem that a photosensitizer does not function adequately, and when an irradiation to a large area is performed, a long time irradiation is required.

Further, there are cases in which a xenon lamp or halogen lamp is used.

However, the emission spectrum of a xenon lamp or halogen lamp covers a wide range of wavelengths continuously. On the other hand, the wavelength region of absorption of a photosensitizer is relatively narrow. Thus, a xenon lamp or halogen lamp is not practical, due to the fact that medical treatment efficacy is low and energy efficiency is poor, relative to the high consumption of electric power.

Furthermore, a xenon lamp and a halogen lamp contain large amounts of lights of wavelength regions (600nm or less, and 800nm or more), which only reach the extreme surface of the tissue of the body or does not work for the purpose. Therefore, there arises a problem that, even if this kind of lights are cut off by a filter or the like, a part of the unnecessary radiating lights is irradiated directly on an affected part, and which thereby causes a patient to feel a sensation of heat.

### SUMMARY OF THE INVENTION

An object of the present invention is to provide a discharge lamp, which mainly radiates a light suitable to a photosensitizer to be used in PDT and PDD, and, in addition, which does not radiate a light other than the light.

A discharge lamp of the present invention radiates a light suitable for the wavelength range of absorption of a photosensitizer having a relatively large absorption coefficient within the region of the wavelength range of 600nm-800nm.

More specifically, as an emitting element, a discharge lamp of the present invention is filled with 0.1 *µ* mol/cm³ or more of any element selected from the group of lithium (Li), sodium (Na), rubidium (Rb), and potassium (K). Furthermore, the discharge lamp is also filled with a gas of at least one selected from the group of rare gases of neon (Ne), argon (Ar), krypton (Kr), and xenon (Xe).

By filling the discharge lamp with the emitting elements, it is possible for the discharge lamp to preferably radiate a light having a wavelength region of the main absorption within the range of the wavelength of 600nm-800nm.

Here, a wavelength region of the main absorption refers to a wavelength region, which is radiated from a discharge lamp and has enough intensity to be used practically. And, the wavelength region of the main absorption is formed within at least one part in the region of the wavelength of 600nm-800nm.

Furthermore, the present invention, specifically, is characterized in that a discharge lamp is filled with lithium (Li) as the emitting element for radiating the lights of 600nm-640nm and 660nm-720nm as the wavelength region of the main absorption.

Further, the present invention is characterized in that a discharge lamp is filled with sodium (Na) as the emitting element for radiating a light of 600nm-640nm as the wavelength region of the main absorption.

Further, the present invention is characterized in that a discharge lamp is filled with rubidium (Rb) as the emitting element for radiating a light of 760nm-800nm as the wavelength region of the main absorption.

Further, the present invention is characterized in that a discharge lamp is filled with potassium (K) as the emitting element for radiating a light of 760nm-800nm as the wavelength region of the main absorption.

Further, the present invention is characterized in that a discharge lamp is filled with at least two or more selected from the group of lithium (Li), sodium (Na), rubidium (Rb), and potassium (K) as emitting elements.

Further, the present invention is characterized in that, in addition to the above-mentioned emitting elements, a discharge lamp is filled with mercury. This is to adjust the voltage of a discharge lamp and increase the radiation within the range of the wavelength of 600nm-800nm resulting from the above-mentioned emitting element.

Furthermore, a discharge lamp of the present invention, which is used in photodynamic therapy or photodynamic diagnosis, preferably radiates both lights within the range of the wavelength (600nm-800nm) suitable to a photosensitizer, and the range of the wavelength (400nm-440nm) suitable for generating fluorescence.

In this place, in addition to filling a discharge lamp with 0.1 *µ* mol/cm³ or more of lithium (Li), sodium (Na), rubidium (Rb), or potassium (K) as a emitting element, the present invention is characterized in that the discharge lamp is filled with 0.1 *µ* mol/cm³ or more of mercury.

By so doing, the discharge lamp can radiates a light of the wavelength region of the main absorption within the range of the above-mentioned wavelength.

Specifically, by filling a discharge lamp with lithium (Li) as an emitting element, it is possible for the discharge lamp to radiate the lights of 600nm-640nm and 660nm-720nm as the wavelength region of the main absorption, and in addition thereto, by filling the discharge lamp with mercury, it is possible for the discharge lamp to radiate the lights of 400nm-410nm and 430nm-440nm as the wavelength region of the main absorption.

Further, by filling a discharge lamp with sodium (Na) as an emitting element, it is possible for the discharge lamp to radiate the light of 600nm-700nm as the wavelength region of the main absorption, and in addition thereto, by filling the discharge lamp with mercury, it is possible for the discharge lamp to radiate the lights of 400nm-410nm and 430nm-440nm as the wavelength region of the main absorption.

Further, by filling a discharge lamp with rubidium (Rb) as an emitting element, it is possible for the discharge lamp to radiate the light of 755nm-800nm as the wavelength region of the main absorption, and in addition thereto, by filling the discharge lamp with mercury, it is possible for the discharge lamp to radiate the lights of 400nm-410nm and 430nm-440nm as the wavelength region of the main absorption.

Further, by filling a discharge lamp with potassium (K) as an emitting element, it is possible for the discharge lamp to radiate the light of 700nm-800nm as the wavelength region of the main absorption, and in addition thereto, by filling the discharge lamp with mercury, it is possible for the discharge lamp to radiate the lights of 400nm-410nm and 430nm-440nm as the wavelength region of the main absorption.

Further, the present invention is characterized in that a discharge lamp is filled with at least two or more of lithium (Li), sodium (Na), rubidium (Rb) or potassium (K) as emitting elements for radiating a light of the range of the wavelength of 600nm-800nm as the wavelength region of the main absorption.

Furthermore, the present invention is characterized in that, in addition to the alkali metals of lithium (Li), sodium (Na), rubidium (Rb) and potassium (K), mercury, and rare gases, halogen is filled into a discharge lamp used in photodynamic therapy and/or photodynamic diagnosis.

### BRIEF DESCRIPTION OF THE DRAWINGS

Fig. 1 is a sketch of an irradiation apparatus equipped with a lamp for PDT.
Fig. 2 is a schematic diagram showing the constitution of a discharge lamp of the present invention.
Fig. 3 is a diagram showing an emission spectrum of a discharge lamp into which Li is filled of the present invention.
Fig. 4 is a diagram showing an emission spectrum of a discharge lamp into which Na is filled of the present invention.
Fig. 5 is a diagram showing an emission spectrum of a discharge lamp into which Rb is filled of the present invention.
Fig. 6 is a diagram showing an emission spectrum of a discharge lamp into which K is filled of the present invention.
Fig. 7 is a diagram showing a radiant spectrum of a light radiated from an irradiation apparatus of the present invention.
Fig. 8 is a diagram showing a radiant spectrum of a light radiated from an irradiation apparatus of the present invention.

### Reference numerals in the Figures

- 1: Lighting housing
- 2: Lamp
- 3: Mirror
- 4: Power source
- 5: Cooling fan
- 6: Filter for cutting wavelength
- 7: Light fiber
- 8: Lens head
- 11: Emitting tube
- 12: Electrodes
- 13: Molybdenum foil
- 14: Outer lead rod

### DESCRIPTION OF THE PREFERRED EMBODIMENTS

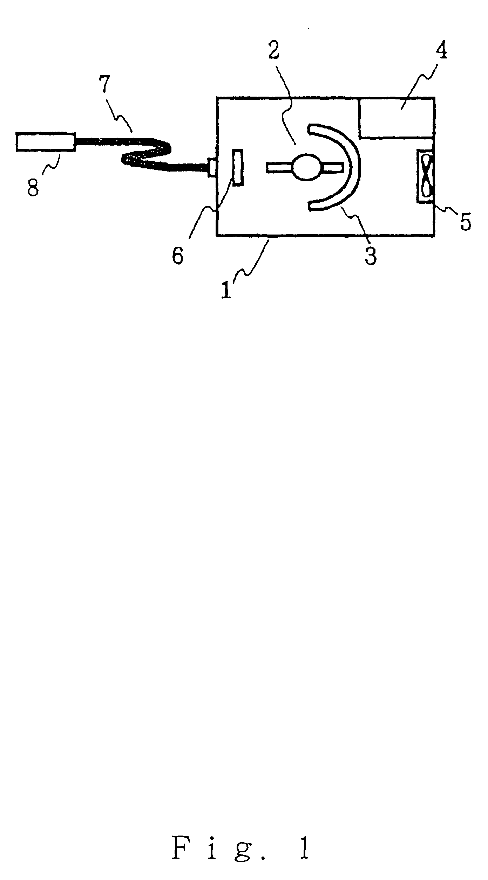

Fig. 1 shows an irradiation apparatus, in which a discharge lamp of this invention is used.

Inside a lamp housing 1, a discharge lamp 2, a mirror 3, which surrounds the discharge lamp, and a power source 4 for lighting the discharge lamp are arranged. A fan 5 is provided on the wall of the lamp housing 1, and the discharge lamp 2 and mirror 3 are cooled by the fan.

Light radiated from the lamp 2 can be incident on an optical fiber 7 via a filter 6. After being incident on the optical fiber 7, the light is irradiated on an affected part from a lens head 8 mounted on the other end of the fiber 7.

Here, the discharge lamp 2 is a short arc-type discharge lamp having the wavelength region of the main absorption (main radiation spectrum) within the range of the wavelength of 600nm-800nm.

The filter 6 has a function for cutting out unnecessary light, and, as a filter for PDT, the filter which cuts out light of the wavelength greater than 800nm and light of the wavelength less than 600nm is used, and as a filter for PDD, the filter which transmits only light in the vicinity of 405nm, and cuts out lights of the shorter side and the longer side of wavelength thereof is used.

This filter 6 is selected according to an application thereof and a type of a photosensitizer. Light transmitted through a filter is applied to a photosensitizer, for example, 5-aminolevulinic acid or Photoprin, which will be referred hereinafter.

Furthermore, in this embodiment, a transmission-type filter is used, but in the present invention, the filter as a means for selecting a wavelength is not limited thereto. For example, a filter coated with an interference film can be used as a reflecting mirror, and other optical components can be used.

Furthermore, a discharge lamp is not limited to a short-arc type. For example, a long-arc-type discharge lamp, or an electrodeless-type discharge lamp can also be employed.

For example, in the case of performing a medical treatment by using an optical fiber, a short-arc-type discharge lamp capable of highly condensing light is preferably used, and in the case of treating an external affected part on the skin, a long-arc-type discharge lamp capable of irradiating a large area thereof is preferably used.

Further, a lighting system of the lamp in the present invention is not limited to either direct-current lighting or alternating-current lighting.

Then, as typical photosensitizer, there exist hematoporphyrin derivatives, phthalocyanine systems, and 5-aminolevulinic acid (hereinafter referred to as 5-ALA) systems.

There are cases that some of these photosensitizers produce various side affects, and thereby cause a great burden to a patient being administered with the agent. For example, since a hematoporphyrin derivative administered is naturally discharged from the body during several weeks after being administered, the patient administered with the agent must stay in a darkened room for around several weeks following the administration thereof, in order to shield an affected part, in which the photosensitizer has accumulated, from the light.

As results of recent researches, photosensitizers having few side effects have been developed, and as a photosensitizer that is naturally discharged from the body during a short period of time after being administered, there exist 5-ALA and the like. Further, as photosensitizers, photosensitizers having no toxicity or side effects on a human body, or having mild toxicity is required. Furthermore, it is preferable to use a photosensitizer which selectively accumulates in the cells of a cancer or tumor, and which, when a light is irradiated on a photosensitizer accumulated in the cells of a cancer or the like, generates active types photochemically with high quantum efficiency.

These photosensitizers have a peculiar photosensitive wavelength, and possess a relatively large absorption coefficient in a specific wavelength region.

For a medical treatment, a light within the range of the wavelength of 600nm-800nm is used for the sake of being able to centrally permeate into the above-mentioned tissue, and for a diagnosis, a light of the range of the wavelength of 400nm-440nm is used for the sake of causing a photosensitizer to fluoresce.

And as photosensitizers that possess a relatively large absorption coefficient in the range of wavelength of 600nm-800nm, for example, there exist Hematoporphyrin derivatives (Photophrin, etc.), 5-ALA (5-aminolevulinic acid), and the 5-ALA derivatives Pp IX (Protoporphyrin IX), Pheophorbide a, ALPcS4 (Aluminum phthalocyanine tetrasulphate), SnET2 (Tin etiopurpurin) , ZnOPPc (Zinc(II)-octadecyl-phthalocyanine), Purprinimide, Azachlorin, ZnET2 (Zinc etiopurpurin), Pc (Phthalocyanine), CdTX (Cd²⁺ texaphyrin), texaphyrin, ZnTNP (Zn-Tetraphptaloporphyrin), Verdin, BPD-MA (Benzoporphyrin derivative monoacid ring A), Purprin, ZnTSPc, Ga-Pc (Ga-Phthalocyanine), In-Pc (In-Phthalocyanine), BPD (Benzoporphyrin derivatives), CASPc, ZnPc (Zinc Phthalocyanine derivatives), AlSPc (Aluminum sulphonated Phthalocyanine), Benzoporphy derivatives, Npe6 (N-aspartyl chlorin e6), Methylene blue, Verteporfin, Rhodamines, Temoporfin, Porphycenes, Hypercin and so forth.

These photosensitizers are well suitable to the range of the wavelength of 600nm-800nm, and it has been discovered that alkali metals such as lithium, sodium, rubidium, potassium and the like are suitable as elements having a strong emission spectrum, i.e. the wavelength region of the main absorption, within the range of the wavelength thereof.

In this place, as for the wavelength region of the main absorption resulting from the elements thereof, it is not necessary for an element to emit a light across the entire range of the wavelength (600nm-800nm), suitable to the above-mentioned photosensitizers, but rather, it is sufficient for an element to emit a light with enough intensity to be used practically in at least a part within the range thereof.

And, according to the present invention, it has been discovered that, in a discharge lamp to be used in photodynamic therapy and a discharge lamp to be used in photodynamic diagnosis, it is effective to fill the discharge lamp with 0.1 *µ* mol/cm³ or more of lithium, sodium, rubidium, potassium, or a mixture thereof as an emitting element for providing a lamp having a wavelength region of the main absorption in the range of the wavelength suitable to a photosensitizer.

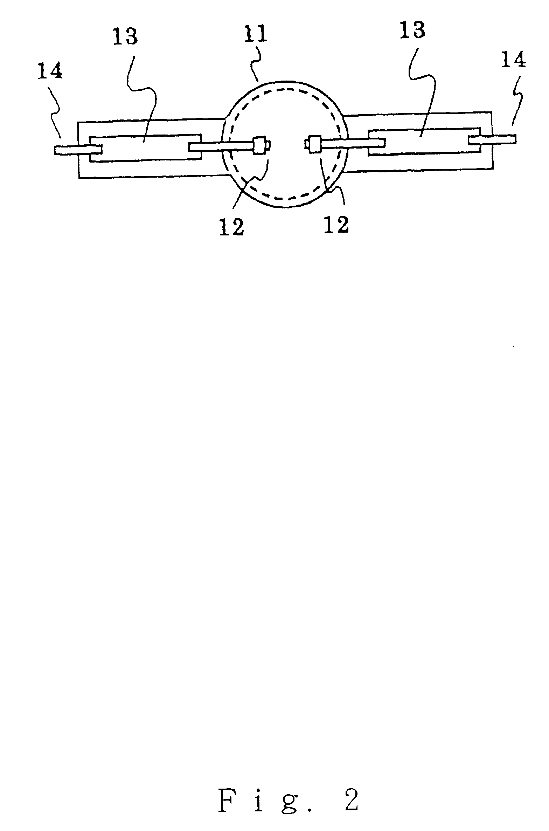

Fig. 2 shows a discharge lamp of the present invention.

The discharge lamp has, for example, a simplified spherical-shaped quartz glass arc tube 11 with an inner diameter of 8mm and an outer diameter of 10.5mm, and has a pair of electrodes 12 inside the arc tube 11 thereof. It is preferable that the distance between electrodes in the electrodes 12 is 10mm or less. The electrodes 12 are welded to molybdenum foil 13, and are sealed at both ends of the arc tube 11. Electric current is applied from outside via outside lead rods 14 which is welded to the molybdenum foils 13 thereof.

An alkali metal such as lithium, sodium, rubidium, potassium or the like is filled inside the arc tube 11 as an emitting element. Further, a rare gas is also filled, and, in addition, mercury is filled as the need arises.

Then, a discharge lamp to be used in photodynamic therapy (PDT) is characterized in that the discharge lamp is filled with lithium, sodium, rubidium, potassium or a mixture thereof as an emitting element.

By filling a discharge lamp with such an emitting element, it is possible to radiate a light having a strong emission spectrum (wavelength region of the main emission) in the range of the wavelength of 600nm-800nm, which are the wavelength range of absorption of photosensitizers.

Furthermore, it is possible to fill a discharge lamp with at least two or more of the lithium, sodium, rubidium, or potassium thereof. By filling two or more kinds of alkali metals like this, it is possible to spread emission in the range of the wavelength of 600nm-800nm in comparison with emission generated by filling only one kind of the alkali metals, and thereby it is possible to increase the kinds of photosensitizers to be used.

Then, a case in which lithium is filled as the emitting element will be explained.

Lithium has intense lines in the emission spectrums between 600nm-640nm and 660nm-720nm. This is the result of the spectral lines in which the atomic spectral line in the wavelength of 610nm, generated as an emission of the lithium, and the atomic resonance spectral line in the wavelength of 670nm are spread by pressure. In particular, the range of the wavelength of the latter (660nm-720nm) is the result of the marked spread of the spectral line in which the atomic resonance spectral line is spread due to the high pressure of a rare gas generated by lighting, and by spreading the spectral width thereof, the selection range of the photosensitizers to be applied are also spread.

Here, the above-mentioned radiation wavelengths generated by using lithium as the emitting element, that is, 600nm-640nm and 660nm-720nm, are equivalent to what is referred to in the present invention as the "wavelength region of the main radiation."

Such a phenomenon can also be seen with alkali metals other than lithium, that is, sodium, rubidium and potassium. The wavelength region of the main radiation in the case of sodium is 600nm-640nm, the wavelength region of the main radiation in the case of rubidium is 755nm-800nm, and the wavelength region of the main radiation in the case of potassium is 760nm-800nm.

Further, the intensity of radiation of the line in the emission spectrum of an alkali metal (emitting element) thereof is determined by the quantity of alkali metal filled inside a lamp. If the quantity of an alkali metal filled is low, the intensity of radiation of the line in the emission spectrum decreases, and the intensity of radiation increases in line with increasing the quantity of an alkali metal filled. However, if an alkali metal as an emitting element is filled in excess of the threshold value, by contrast, the intensity of radiation of the line in the emission spectrum thereof incurs a large decrease by self-absorption. Furthermore, the emitting element does not vaporize inside the lamp, and, as a result, causes the adherence thereof to the walls of the tube, and thereby the intensity of radiation from the lamp is decreased.

In the present invention, the threshold value of an emitting element to be used is 100 *µ* mol/cm³, and if the value of the emitting element to be used exceeds the above value, the above-mentioned phenomenon occurs. Further, if the quantity of an emitting element to be filled is less than 0.1 *µ* mol/cm³, it is not possible to achieve enough radiation quantities to be absorbed preferably by a photosensitizer.

In other words, the quantity of lithium, sodium, rubidium or potassium to be filled as an emitting element must be between 0.1-100 *µ* mol/cm³.

Further, when two or more kinds of elements are filled, 0.1 *µ* mol/cm³ or more of each emitting element must be filled. However, the upper limit of 100 *µ* mol/cm³ is considered in accordance with the total value of the elements filled.

Furthermore, a discharge lamp of the present invention is filled with a rare gas as a starting gas.

The rare gas thereof is argon, neon, krypton or xenon, and the quantity thereof to be filled is preferably from 0.03 x 10⁵ Pa to 0.7 x 10⁵ Pa.

If the quantity of a rare gas filled is less than 0.03 x 10⁵ Pa, it takes time for the transition from the glow discharge at lighting of a lamp to arc discharge, and as a result thereof, the electrodes is damaged terribly. Further, if the quantity of a rare gas filled reaches more than 0.7 x 10⁵ Pa, the starting abilities of a lamp becomes impractical.

Furthermore, in a discharge lamp of the present invention, a rare gas has not only functions to start lighting of the lamp like this, but also has the function to increase a line in the emission spectrum of an alkali metal via the pressure of a rare gas filled while a lamp is lit.

Next, a case in which a discharge lamp is filled with mercury in addition to the emitting element of lithium or the like will be explained.

In the present invention, an alkali metal such as lithium, sodium, rubidium or potassium is used as an emitting element as described above, it has been discovered that, by filling a discharge lamp with mercury in addition thereto, it is possible to provide a more useful PDT discharge lamp.

This is the result of that, due to the high pressure of the mercury at lighting of the lamp, it is possible to increase the line in the emission spectrum of an alkali metal. In other words, it is possible to increase the line in the emission spectrum of an alkali metal via the pressure of a rare gas without mercury, but by adding mercury, it is possible to increase the line in the emission spectrum even more. Specifically, it is possible to increase the radiation wavelength to the vicinity of 800nm by filling a discharge lamp with mercury, and thereby the absorption by a photosensitizer can be increased in accordance therewith.

This phenomenon occurs when any of lithium, sodium, rubidium or potassium is used as an emitting element.

However, by adding mercury to a discharge lamp together with the alkali metals thereof, the emissions at wavelengths of 400-410nm and 430-440nm are achieved in accordance with the mercury. The wavelengths thereof are capable of causing a photosensitizer to fluoresce, and in accordance therewith, make it possible to be used in a diagnosis, which enables the location of a cancer or tumor to be confirmed, that is, to be used in a PDD.

Further, by adding mercury, it is possible to adjust the operating voltage of a lamp. Furthermore, by adding mercury, it is possible to prevent the electrodes from being flowed by a high current which shortens lamp life. At the same time, the decrease of the current thereof enables the stabilizer to be made smaller, and in turn, can contribute toward making the apparatus smaller in size.

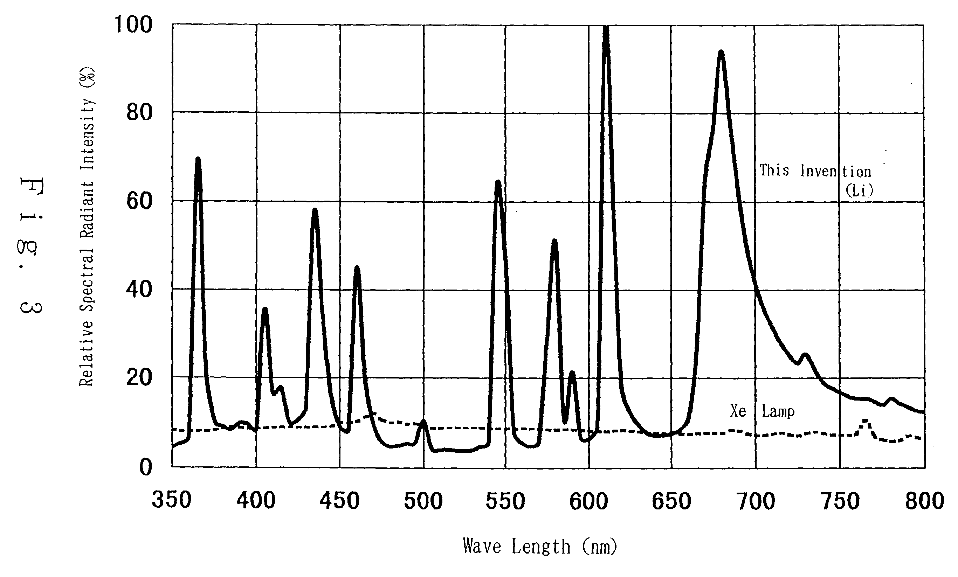

Fig. 3 is a diagram showing the spectral radiant intensity of a discharge lamp filled with lithium and mercury, as compared to the spectral radiant intensity of a xenon lamp.

The horizontal axis shows the wavelength of light radiated from the lamp, and shows a range of 350nm-800nm. Further, the vertical axis shows the highest spectral intensity in the wavelength range of the lithium-mercury lamp in terms of a relative intensity of up to 100%. Furthermore, with regard to the output of a xenon lamp, a relative radiant intensity, having the spectral radiant intensity of the lithium-mercury lamp as a reference, is shown.

Both the lithium-mercury lamp and the xenon lamp used for comparison were operated at an input power of 150W.

Furthermore, it is clear that the radiation of the lithium-mercury discharge lamp are in the range of the wavelengths of 600nm-640nm and 660nm-800nm, and compared to a discharge lamp filled only with lithium without being filled with mercury, the greatest radiation is achieved in the range of the wavelength of 720nm-800nm.

Thus, the xenon lamp emits a light of the continuous range of the wavelength of 350nm-800nm. On the other hand, a lithium-mercury lamp of the present invention has a plurality of lines in the emission spectrum. And, even though the same electric power is inputted, the lithium-mercury lamp of the present invention has lines in the emission spectrum in a range of the wavelength region of absorption of photosensitizer, and, in addition, the spectral radiant intensity thereof are extremely high. It is clear from the characteristics of such radiant intensity thereof that a lithium-mercury lamp of the present invention is a more suitable light source for PDT than a conventional xenon lamp. The radiant intensity of a lithium-mercury lamp at a wavelength of 610nm, for example, is roughly 10-times as compared with that of a xenon lamp.

When the lithium-mercury lamp thereof is preferably used as a light source for PDT, because the atomic line of the wavelength of 610nm due to an emission of lithium, and the resonance line of the wavelength of 670nm are spread by pressure to generate wide spectral lines. Further, when said lamp is used as a light source for PDD, the light required to obtain fluorescence of a photosensitizer can be generated by the atomic line of the wavelength of 404nm in accordance with the mercury. The radiant intensity of the line in the emission spectrum of the wavelength of 404nm thereof is approximately four times as compared with that of a xenon lamp inputted with the same power.

Further, the emitting intensity of the wavelength of 404nm, which is used for PDD, can also be heightened by increasing the quantity of mercury filled.

Thus, a mercury-filled discharge lamp can be used not only for PDT, but for PDD as well, and by providing means, such as a filter, for switching a wavelength to be radiated, can be used for both PDT and PDD.

When said lamp is used for both PDT and PDD, the quantity of mercury to be filled is the above-mentioned 0.1 *µ* mol/cm³ or greater, and preferably, 1 *µ* mol/cm³ or more, and thereby the applicable range of the wavelength is spread even further.

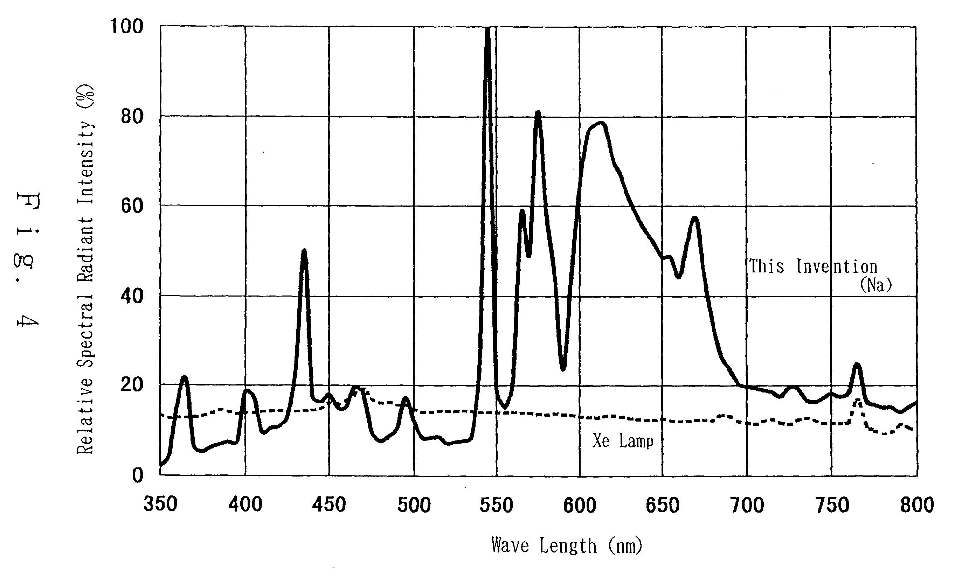

Fig. 4 shows the spectral intensity of a discharge lamp filled with sodium and mercury. The electric power inputted to the sodium-mercury lamp is 150W, the same as that of the above-mentioned embodiment, and the spectral intensity of a xenon lamp inputted with the same power is also shown for a comparison. The vertical axis and horizontal axis are the same as those in the case of the above-mentioned embodiment.

As is clear from the diagram, a sodium-mercury lamp has higher radiant intensity at wavelength of 600nm-700nm as compared with that of a xenon lamp. Light of this wavelength range, as mentioned above, is useful as a light source for PDT. Furthermore, it is also clear that output thereof is higher than that of a xenon lamp in the range of 750nm-800nm.

Output thereof in the range of the wavelengths of 750nm-800nm is changed according to the quantities of mercury and sodium filled. Further, a sodium-mercury lamp also has higher line in the emission spectrum as compared with that of a xenon lamp in the range of the wavelength of 400nm-440nm, and is used preferably in the range of wavelength thereof as a light source for PDD as well.

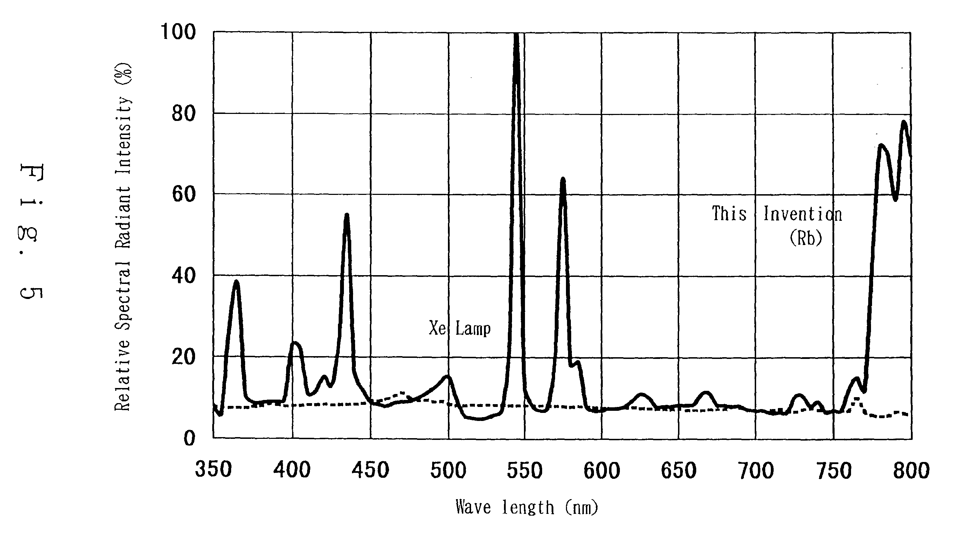

Fig. 5 shows the spectral radiant intensity of a discharge lamp filled with rubidium and mercury. The vertical axis and horizontal axis are the same as those in the case of the above-mentioned embodiment, and the electric power inputted to the discharge lamp is 150W, the same as that of the above-mentioned embodiment. The radiant intensity of a xenon lamp inputted with the same power is also shown for a comparison.

It is clear from the diagram that the rubidium-mercury lamp has higher output thereof as compared with that of a xenon lamp at wavelength of 750nm-800nm, and it is also clear that the rubidium-mercury lamp has a higher line in the emission spectrum at wavelengths of 400nm-440nm. The light of the former wavelength range can be used as a light source useful for PDT, and the light of the latter wavelength range can be used as a light source useful for PDD.

Furthermore, the output of the former wavelength range can be changed in accordance with the quantities of mercury and rubidium filled.

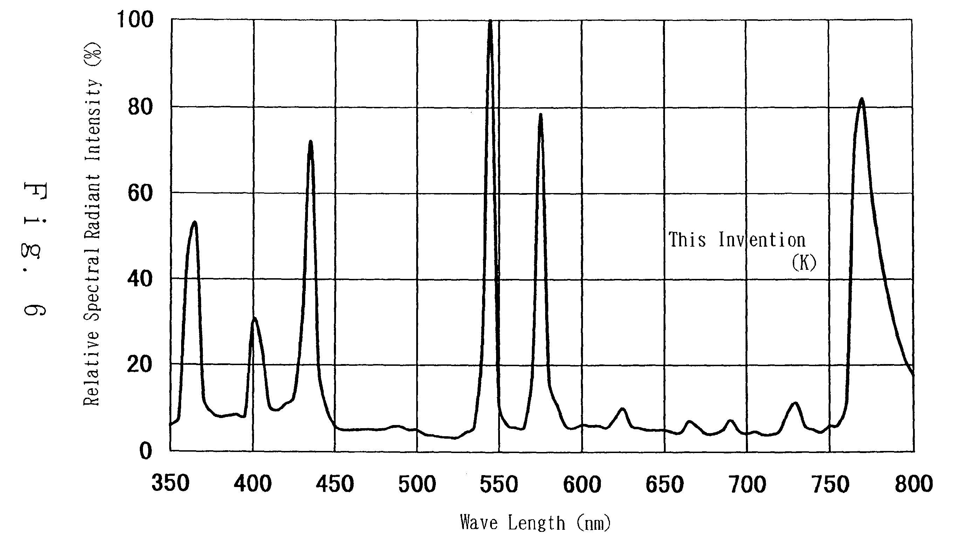

Fig. 6 shows the spectral radiant intensity of a discharge lamp filled with potassium and mercury. The vertical axis and horizontal axis are the same as those in the case of the above-mentioned embodiment, and the electric power inputted to the discharge lamp is 150W, the same as that of the above-mentioned embodiment. The radiant intensity of a xenon lamp inputted with the same power is also shown for a comparison.

It is clear from the diagram that the potassium-mercury lamp has higher output as compared with that of a xenon lamp at wavelength of 750nm-800nm, and it is also clear that the potassium-mercury lamp has a higher line in the emission spectrum at wavelength of 400nm-440nm. The light of the former wavelength range can be used as a light source useful for PDT, and the light of the latter wavelength range can be used as a light source useful for PDD.

Furthermore, the output of the former wavelength range can be changed in accordance with the quantities of mercury and potassium to be filled. In the above-mentioned discharge lamps filled with mercury and each of lithium, sodium, rubidium, or potassium, the quantity of mercury to be filled is 0.1 *µ* mol/cm³-1000 *µ* mol/cm³.

This is because, if the quantity of mercury filled is less than 0.1 *µ* mol/cm³, emitting light sufficiently for being absorbed by a photosensitizer is not obtained.

Further, if the quantity of mercury filled is greater than 1000 *µ* mol/cm³, there exists a danger of either causing the mercury not to vaporize, or causing the arc tube to bread due to a high filling pressure during lighting.

Thus, the quantity of mercury to be filled is preferably selected within the range of 0.1 *µ* mol/cm³-1000 *µ* mol/cm³ based on the amount of light emitted from a lamp and the voltage thereof.

Further, in the above-mentioned embodiments, only one kind of alkali metal is filled into each discharge lamp, but two or more kinds of the alkali metals thereof can be filled.

That is, at least two or more kinds of elements selected from the group of lithium (Li), sodium (Na), rubidium (Rb) and potassium (K) can be filled, and, in accordance therewith, the emitting light at wavelengths of 600nm-800nm can be spread more as compared with a case in which one kind of element is filled. Thus, it is possible to increase the kinds of photosensitizer to be used.

Further, in the above-mentioned discharge lamp to be filled with mercury, at least one or more kinds of rare gases selected from the group of neon (Ne), argon (Ar), krypton (Kr) and xenon (Xe) can be filled as a starting gas. In accordance therewith, the starting ability of a lamp is enhanced, and, in addition, the line in the emission spectrum of an alkali metal can be spread by the rare gases thereof.

Then, the material of an arc tube utilized in a discharge lamp will be explained.

As an arc tube material, in general, quartz glass is often used. However, in the case of a discharge lamp to be filled with an alkali metal like a discharge lamp of the present invention, a quartz glass is corroded by the alkali metals thereof, and, thereby there occurs problem, that transmittance thereof is degradated or cloudiness thereof is caused.

Thus, in a discharge lamp of the present invention, a translucent ceramic is preferably used as an arc tube material.

However, if an alkali metal such as lithium, or a mixture thereof is used not as a metal but as a halogenated configuration, quartz glass can also be used as the material for an arc tube.

It is preferable to prepare the filling quantity of halogen in this case as same or greater than the filling molecular quantity in the case of an alkali metal of lithium or the like.

A halide, more specifically, can be filled in the form of an iodide or bromide. Furthermore, when the occasion demands, surplus halogen can be filled, for example, surplus halogen can be filled in the form of mercury iodide.

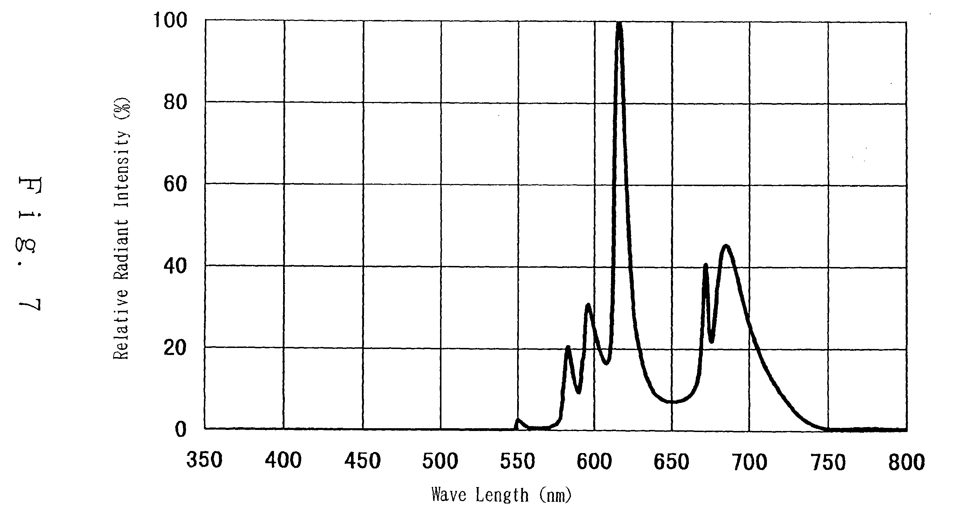

Fig. 7 shows the spectrum of light outputted from an irradiation apparatus. Specifically, this figure shows the wavelength of radiated light which is radiated from a lithium-mercury discharge via a filter, optical fiber and a lens head. In this embodiment, since emphasis is placed on the range of the wavelength of 600nm-700nm, light of 750nm-800nm is cut off by a filter.

And, radiant illuminance measured at a location roughly 40mm away from the lens head was approximately 65mW/cm².

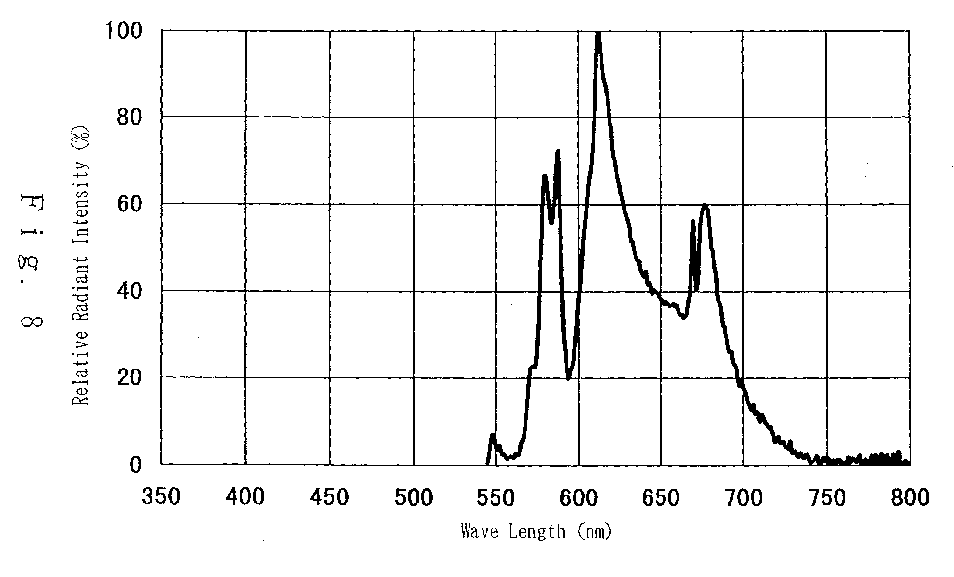

Fig. 8 shows the spectral spectrum of light outputted from an irradiation apparatus, in which a sodium-mercury discharge lamp is used. In this embodiment, light that does not contain the light outside the range of wavelength of 550nm-750nm which is cut out by a filter is irradiated from a lens head via an optical fiber.

It is clear from the figure that light of the range of wavelength of 550nm-750nm is radiated.

Table 1 depicts data of examples of medical treatments by PDD using a lithium-mercury discharge lamp.

**Table 1**

| Classifications | Number thereof |
|---|---|
| Subjects tested | 9 |
| Subjects cured | 6 |
| Subjects improved | 3 |

In the experiment, an ointment mixed with 5-ALA as a photosensitizer was applied topically to each of 9 subjects to be tested having early-stage skin cancer, and radiating light from a lithium-mercury lamp was irradiated on an affected part thereof.

As a result thereof, it was confirmed that 6 of the 9 subjects were cured in accordance with irradiations of around 1-7 times, and the condition of remaining 3 subjects were improved preferably.

As explained above, the present invention can provide a photodynamic therapy (PDT) discharge lamp, and a photodynamic diagnosis (PDD) discharge lamp, which effectively emits light toward a photosensitizer having a specific photosensitive wavelength, said light is suitable for the photosensitive wavelength thereof.

Further, a discharge lamp of the present invention is capable of achieving higher radiant efficiency as compared with that of a xenon lamp at relatively low power consumption, and thereby the size of the entire apparatus including the power source can be reduced.

Furthermore, the light thereof irradiated on a normal tissue of the body is safer as compared with that of a laser or the like, and is operated simply.

Furthermore, a lamp which is a long arc-type discharge lamp can irradiate a large area of skin from the outside, and thereby the burden on a patient can be reduced.

## Claims

1. A discharge lamp for photodynamic therapy comprising,
a discharge lamp which radiates a light of a wavelength suitable to the wavelength range of absorption of a photosensitizer having a relatively large absorption coefficient within the range of the wavelengths of 600nm-800nm, and
has a function to emit light having the wavelength region of the main absorption within the range of the wavelengths of 600nm-800nm,
said discharge lamp is filled with 0.1 *µ* mol/cm³ or more of any selected from the group of lithium (Li), sodium (Na), rubidium (Rb), or potassium (K) as an emitting element, and
further filled with at least one or more of rare gases selected from the group of neon (Ne), argon (Ar), krypton (Kr) and xenon (Xe).

2. The discharge lamp for photodynamic therapy of Claim 1, wherein lithium (Li) is filled as the emitting element for radiating the lights of 600nm-640nm, and 660nm-720nm of the wavelength region of the main absorption.

3. The discharge lamp for photodynamic therapy of Claim 1, wherein sodium (Na) is filled as the emitting element for radiating the light of 600nm-640nm of the wavelength region of the main absorption.

4. The discharge lamp for photodynamic therapy of Claim 1, wherein rubidium (Rb) is filled as the emitting element for radiating the light of 755nm-800nm of the wavelength region of the main absorption.

5. The discharge lamp for photodynamic therapy of Claim 1, wherein potassium (K) is filled as the emitting element for radiating the light of 760nm-800nm of the wavelength region of the main absorption.

6. The discharge lamp for photodynamic therapy of Claim 1, wherein at least two or more elements selected from the group of lithium (Li), sodium (Na), rubidium (Rb) and potassium (K) are filled as the emitting elements.

7. The discharge lamp for photodynamic therapy of Claim 1, wherein mercury (Hg) is further filled for increasing line in the emission spectrum of said lithium (Li), sodium (Na), rubidium (Rb), potassium (K).

8. A discharge lamp for photodynamic therapy or photodynamic diagnosis comprising,
a discharge lamp which radiates a light of a wavelength suitable to the wavelength range of absorption of a photosensitizer having a relatively large absorption coefficient within the range of the wavelength of 600nm-800nm, and a light of a wavelength suitable to a photosensitizer, which absorbs light within the range of the wavelength of 400nm-440nm, and emits fluorescence, and
has a function to emit light having the wavelength region of the main absorption within the range of the wavelength of 600nm-800nm and also emit light having the wavelength region of the main absorption within the range of the wavelength of 400nm-440nm,
said discharge lamp is filled with 0.1 *µ* mol/cm³ or more of any selected from the group of lithium (Li), sodium (Na), rubidium (Rb), or potassium (K), and 0.1 *µ* mol/cm³ or more of mercury (Hg) as an emitting element, and
further filled with at least one or more rare gases selected from the group of neon (Ne), argon (Ar), krypton (Kr) and xenon (Xe).

9. The discharge lamp for photodynamic therapy or photodynamic diagnosis of Claim 8, wherein lithium (Li) is filled as the emitting element for radiating the lights of 600nm-640nm, and 660nm-800nm of the wavelength region of the main absorption.

10. The discharge lamp for photodynamic therapy or photodynamic diagnosis of Claim 8, wherein sodium (Na) is filled as the emitting element for radiating the light of 600nm-700nm of the wavelength region of the main absorption.

11. The discharge lamp for photodynamic therapy or photodynamic diagnosis of Claim 8, wherein rubidium (Rb) is filled as the emitting element for radiating the light of 755nm-800nm of the wavelength region of the main absorption.

12. The discharge lamp for photodynamic therapy or photodynamic diagnosis of Claim 8, wherein potassium (K) is filled as the emitting element for radiating the light of 760nm-800nm of the wavelength region of the main absorption.

13. The discharge lamp for photodynamic therapy or photodynamic diagnosis of Claim 8, wherein at least two kinds or more selected from the group of lithium (Li), sodium (Na), rubidium (Rb) and potassium (K) are filled as the emitting elements.

14. The discharge lamp for photodynamic therapy of Claim 1, wherein halogen is also filled into said discharge lamp.

15. The discharge lamp for photodynamic therapy or photodynamic diagnosis of Claim 8, wherein halogen is also filled into said discharge lamp.
